# EUROPEAN PATENT APPLICATION

(11) **EP 3 326 653 A1**
(43) Date of publication of application: **30.05.2018**
(21) Application number: 17203118.9
(22) Date of filing: 22.11.2017
(51) Int. Cl.: A61K 47/32

(54) **DELAYED RELEASE DOSAGE FORMS COMPRISING DIMETHYL FUMARATE**

(30) Priority: 23.11.2016 TR 201616998
(71) Applicant: Sanovel Ilac Sanayi ve Ticaret A.S., 34460 Istanbul (TR)
(72) Inventor: TÜRKYILMAZ, Ali, 34460 Istanbul (TR); KESKIN, Reyhan, 34460 Istanbul (TR); CILEN, Hüseyin, 34460 Istanbul (TR); CELIK, Devrim, 34460 Istanbul (TR); ACAR, Serkan, 34460 Istanbul (TR)
(74) Representative: Sevinç, Erkan

(57) **Abstract**

The present invention is relates to a delayed release pharmaceutical composition comprising dimethyl fumarate and at least one pharmaceutically acceptable excipient.

## Description

### Technical Aspect

The present invention relates to a delayed release pharmaceutical composition comprising dimethyl fumarate and at least one pharmaceutically acceptable excipient.

### Background of the Invention

Multiple sclerosis (MS) is the most common autoimmune disorder affecting the central nervous system. Multiple sclerosis, also known as disseminated sclerosis or encephalomyelitis disseminata, is a demyelinating disease in which the insulating covers of nerve cells in the brain and spinal cord are damaged. This damage disrupts the ability of parts of the nervous system to communicate, resulting in a wide range of signs and symptoms, including physical, mental and sometimes psychiatric problems. MS takes several forms, with new symptoms either occurring in isolated attacks (relapsing forms) or building up over time (progressive forms). Between attacks, symptoms may disappear completely; however, permanent neurological problems often occur, especially as the disease advances.

Dimethyl fumarate (DMF) corresponding to the compound dimethyl (E)- butenedioate (C₆H₈O₄) is an anti-inflammatory mostly used for treating multiple sclerosis (MS). Its chemical structure is shown in the Formula I.

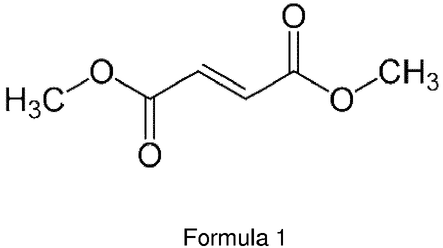

Dimethyl Fumarate is marketed by BIOGEN under the trademark TECFIDERA®. This formulation, TECFIDERA®, is available as hard gelatin delayed-release capsules containing 120 mg or 240 mg of granulated dimethyl fumarate enterically coated minitablets.

There are various patents in the state of the art in relation to dimethyl fumarate which is first described in US6509376 B1 and also discloses its indication.

The patent application WO2015130998 A1 discloses a formulation, comprising fumarate ester as an active agent, lactic acid, polyoxyl 40 hydrogenated castor oil, polyvinylpyrrolidone, mixture of mono and di-glycerides. In particular, oral pharmaceutical compositions comprises controlled release enteric soft capsules. Enteric coat comprises, gelatin, glycerol, methylacrylic acid copolymer, triethyl citrate, ammonia, water and titanium dioxide.

The patent application EP2811994 A4 discloses containing dimethyl fumarate and pharmaceutically acceptable salt thereof that metabolize to monomethyl fumarate with certain pharmacokinetic parameters and methods for treating, prophylaxis, or amelioration of neurodegenerative diseases including multiple sclerosis using such compositions in a subject.

In prior art, dimethyl fumarate has a particle size lower than 250 micron and so occurs the problem of sublimation of dimethyl fumarate. Thus, desired amount of dimethyl fumarate in mini tablet may decrease unexpectedly.

In comparison to prior art, at present invention dimethyl fumarate has a particle size d(0.8) value is greater than 250 micron and d(0.9) value is less than 950 micron in order to decrease the sublimation of dimethyl fumarate and enhance the flowability of the drug product powder. By using dimethyl fumarate having a particle size distribution between this range in the drug powder product, equivalent dissolution profile is provided with the reference product. Furthermore mini tablets are coated with at least one film coating, especially pre-coating, this provides decrease in sublimation and enhance in dissolution profile and mechanical stability.

### Description of the Invention

The main object of the present invention is to provide a formulation with high stability, desired level of dissolution rate and bioavailability which overcomes the above described problems in prior art and have additive advantages over them.

Accordingly, another object of the present invention is to decrease the sublimation of dimethyl fumarate by the help of increasing the particle size of d (0.8) greater than 250 µm. Thus, the surface area of dimethyl fumarate is decreased by increasing the particle size and so overcomes the above described problems in prior art.

Accordingly, another object of the present invention is to provide high chemical and mechanical stability with pre-coating. Also specifically, it has been surprisingly discovered that the pre-coating formulations in the form of a mini-tablet that increase the solubility of dimethyl fumarate.

According to one embodiment, the delayed release pharmaceutical composition comprises dimethyl fumarate and at least one pharmaceutically acceptable excipient wherein the total amount of dimethyl fumarate in the composition is 18% to 70% by weight. Preferably the total amount of dimethyl fumarate in the composition is 30% to 60% by weight, more preferably 35% to 43% by weight.

According to this embodiment, dimethyl fumarate has a particle size d (0.8) greater than 250 µm, preferably it has a particle size d (0.8) 250 µm to 800 µm, more preferably dimethyl fumarate it has a particle size d (0.8) 280 µm to 450 µm.

According to this embodiment, dimethyl fumarate has a particle size d (0.1) 150 µm to 350 µm , preferably it has a particle size d (0.1) 150 µm to 300 µm.

According to this embodiment, dimethyl fumarate has a particle size d (0.9) 800 µm to 1000 µm , preferably it has a particle size d (0.9) 900 µm to 950 µm.

According to this embodiment, dimethyl fumarate has a particle size d (0.1) 255 µm to 300 µm and d (0.8) 280 µm to 450 µm and d (0.9) 900 µm to 950 µm.

As used here in, 'particle size distribution' means the cumulative volume size distrubition as tested by any conventionally accepted method such as the laser diffraction method (i.e. malvern analysis) .The term d(0.1) means, the size at which 10% by volume of the particles are finer and d(0.8) means the size at which 80% by volume of the particles are finer and d (0.9) means the size at which %90 by volume of the particles are finer.

A further embodiment of the invention comprises DMF and at least one excipient, wherein the amount of dimethyl fumarate is 41% by weight and also dimethyl fumarate has a particle size of d(0.8) greater than 250 µm.

According to this invention, the pharmaceutical composition is in the form of a minitablet.

The term "mini tablet", as used herein, refers to small tablets with a diameter equal to or less than 4 mm that are typically filled into a capsule or further compressed into larger tablets. Thickness of this mini tablets equal to or less than 3 mm. The mini tablets have round shape and smooth surface to ease coating process.

According to this invention, minitablets are encapsulated in a capsule comprising 20 minitablets and the amount of dimethyl fumarate is 240 mg per capsule.

According to this invention , the minitablets has at least one coating.

According to this invention , minitablets has at least one film-coating and an enteric coating.

In this present invention, mini tablet is coated with at least one film- coating and enteric coating. The film-coating is applied at two step . First step of film coating is called pre-coating , second step of film-coating is applied after enteric coating. In other words, first film coating (pre-coating) is applied, second enteric coating is applied and last second film coating is applied.

In mini tablets, high solubility for dimethyl fumarate is essential in order to achieve desired content uniformity. Pre-coating increase the solubility of dimethyl fumarate. This helps to achieve desired dissolution profile and bioavailability.

According to one embodiment, the enteric coating layer comprises methacrylic acid/ethylacrylate 1:1 co polymer, talc, titanium dioxide, triethyl citrate or mixtures thereof.

According to this invention , the enteric coating layer comprising 50% to 75% by weight of methacrylic acid/ethylacrylate 1:1 co polymer ,15% to 25% by weight of talc, 5% to 20% by weight of titanium dioxide, 2% to 15% by weight of triethyl citrate or mixtures thereof.

In further embodiment, enteric coating layer comprises , 55% to 70% by weight of methacrylic acid/ethylacrylate 1:1 co polymer , 22% to 17% by weight of talc, 10% to 14% by weight of titanium dioxide, 6% to 10% by weight of triethyl citrate or mixtures thereof.

According to this invention, the film-coating layer comprising hydroxypropylmethyl cellulose , triacetin/glycerol triacetate, talc or mixtures thereof.

According to this invention , the film-coating layer comprising 80% to 90% by weight of hydroxypropylmethyl cellulose , 7% to 10% by weight of triacetin/glycerol triacetate, 6% to 8% by weight of talc or mixtures thereof.

In further embodiment, film-coating layer comprises 82% to 85% by weight of hydroxypropylmethyl cellulose , 8% to 9% by weight of triacetin/glycerol triacetate, 7% to 8% by weight of talc or mixtures thereof.

In further, film-coatings and enteric coating provide high chemical and mechanical stability. Also , coatings especially enteric coating prevent damage to the stomach of the dimethyl fumarate.

In a further embodiment, two different coating is used for dimethyl fumarate desired dissolution. The film-coating helps to provide the desired dissolution profile. Enteric coating provides resistant to acidity of mini tablet until the mini tablet reaches the small intestine. With the help of the enteric coating mini tablets are dissolved in intestine not in stomach. Therefore, desired dissolution can be achieved. Furthermore, it is very important to choose coating agents. Coating agents may be interact with active agents or excipients. However, in this invention it has been found that either HPMC or methacrylate copolymer is not chemically interact with the formulation. They also provide chemical stability for the formulation.

Accordingly a further embodiment of the invention comprises a pharmaceutical composition comprising dimethyl fumarate and at least one fillers, binders, disintegrants , lubricants , glidant or mixtures thereof.

Suitable fillers are selected from group comprising ammonium alginate, calcium carbonate, calcium phosphate, calcium sulfate, cellulose, cellulose acetate, compressible sugar, confectioner's sugar, dextrates, dextrin, dextrose, erythritol, ethylcellulose, fructose, glyceryl palmitostearate, hydrogenated vegetable oil type I, isomalt, kaolin, lactitol, lactose, mannitol, magnesium carbonate, magnesium oxide, maltodextrin, maltose, mannitol, medium chain triglycerides, microcrystalline cellulose, polydextrose, polymethacrylates, polyvinylpyrrolidone, simethicone, sodium alginate, sodium chloride, sorbitol, starch, sucrose, sugar spheres, sulfobutylether beta-cyclodextrin, talc, tragacanth, trehalsoe, polysorbate 80, xylitol or mixtures thereof. Preferably the filler is microcristalline cellulose.

Suitable binders are selected from group comprising ammonium alginate, calcium carbonate, calcium phosphate, calcium sulfate, cellulose, cellulose acetate, compressible sugar, confectioner's sugar, dextrates, dextrin, dextrose, erythritol, ethylcellulose, fructose, glyceryl palmitostearate, hydrogenated vegetable oil type I, isomalt, kaolin, lactitol, lactose, mannitol, magnesium carbonate, magnesium oxide, maltodextrin, maltose, mannitol, medium chain triglycerides, microcrystalline cellulose, polydextrose, polymethacrylates, polyvinylpyrrolidone, simethicone, sodium alginate, sodium chloride, sorbitol, starch, sucrose, sugar spheres, sulfobutylether beta-cyclodextrin, talc, tragacanth, trehalsoe, polysorbate 80, xylitol or mixtures thereof. Preferably the binder is polyvinylpyrrolidone.

In present invention polyvinylpyrrolidone is used as binder because compatible with most organic and inorganic pharmaceutical ingredients and it is appropriate for using delayed release. In addition to these, polyvinylpyrrolidone is used for easy availability and low cost. Also , it increases mechanical stability of the mini tablet.

In one embodiment of the invention the weight ratio of polyvinylpyrrolidone to total weight is 1.0-10.0 , preferably the ratio is 2.0-5.0 .

In one embodiment of the invention, the weight ratio of polyvinylpyrrolidone to weight of dimethyl fumarate is between 0.01-10.0, preferably the ratio is 0.05-5.0 , more preferably the ratio is 0.05-1.0 .

Suitable disintegrants are selected from group comprising hydroxypropyl starch, alginic acid, calcium alginate, carboxymethylcellulose calcium, carboxymethylcellulose sodium, powdered cellulose, chitosan, colloidal silicon dioxide, croscarmellose sodium, crospovidone, docusate sodium, guar gum, hydroxypropyl cellulose, low substituted hydroxypropyl cellulose, magnesium aluminum silicate, methylcellulose, microcrystalline cellulose, polacrilin potassium, povidone, sodium alginate, sodium starch glycolate, starch, and pregelatinized starch or mixtures thereof, preferably the disintegrant is crospovidone.

Suitable glidants are selected from group comprising calcium phosphate, calcium silicate, powdered cellulose, magnesium stearate, magnesium silicate, magnesium trisilicate, silicon dioxide, talcum, colloidal silica and silica colloidal anhydrous or mixtures thereof, preferably the glidant is silica colloidal anhydrous.

Suitable lubricants are selected from group comprising canola oil, hydroxyethyl cellulose, lauric acid, leucine, mineral oil, poloxamers, polyvinyl alcohol, talc, oxtyldodecanol, sodium hyaluronate, sterilizable maize starch, triethanolamine, calcium stearate, magnesium stearate, glycerin monostearate, glyceryl behenate, glyceryl palmitostearate, hydrogenated castor oil, hydrogenated vegetable oil type I, light mineral oil, magnesium lauryl sulfate, medium-chain triglycerides, mineral oil, myristic acid, palmitic acid, poloxamer, polyethylene glycol, potassium benzoate, sodium benzoate, sodium chloride, sodium lauryl sulfate, stearic acid, talc, and zinc stearate or mixtures thereof, preferably the lubricant is magnesium stearate.

One embodiment of the invention is the delayed release pharmaceutical composition comprising between 10% w/w and 95% w/w of DMF as active ingredient ; between 30% w/w and 45% w/w of at least one filler; between 1% and 10% at least one binder; between 0.1% w/w and 2% w%w at least one lubricant; between 0.1% w/w and 2% w%w at least one glidant and between 3% w/w and 10% w%w at least one disintegrant.

Another aspect of the present invention is to provide a process for preparing the pharmaceutical composition, this method comprising the following steps;
(a) mixing dimethyl fumarate, half of microcrystalline cellulose, polyvinylpyrrolidone, colloidal silicon dioxide for 10 minutes;
(b) sieving half of mg stearat and adding the powder mixture of step (a) then mixing for 3 minutes;
(c) dry granulation of the mixed components;
(d) mixing crospovidone and the remaining part of microcrystalline cellulose;
(e) adding this mixture step (d) in step (c) mixture then mixing for 5 minutes;
(f) sieving the remaining part of magnesium stearate and adding it to step (e) mixture then mixing for 3 minutes
(g) compressing the blended mixture to form minitablet
(h)coating with the mini tablet pre-coating, 30%-35% of all coating is pre-coating which comprises hydroxypropylmethyl cellulose, triacetin/glycerol triacetate, talc.
(i) then coating the mini tablet with enteric coating, 45%-55% of all coating is enteric coating which comprises methacrylic acid/ethylacrylate 1:1 co-polymer, talc, titanium dioxide, triethyl citrate.
(j) finally, coating the mini tablets with film coating , 14%-18% of all coating is film-coating which comprises hydroxypropylmethyl cellulose, triacetin/glycerol triacetate, talc,
(k) filling the mini tablets into capsules.

### Example 1

This example describes a delayed release pharmaceutical formulation comprising dimethyl fumarate. The active ingredient and excipients of the tablet are given below:

**Delayed release capsule formulation of dimethyl fumarate**

| **Agents** | **Amount (% by weight of the total)** |
|---|---|
| Dimethyl fumarate | 35% to 43% |
| microcrystalline cellulose | 25% to 45% |
| crospovidone CL | 3% to 10% |
| silica colloidal anhydrous | 0.1% to 3% |
| magnesium stearate | 0.1% to 3% |
| polyvinylpyrrolidone K30 | 2% to 5% |
| Coating | 8% to 12% |

### Example 2

| **Agents** | **Amount (% by weight of the total)** |
|---|---|
| Dimethyl fumarate | 41% |
| microcrystalline cellulose | 37.9% |
| crospovidone CL | 5.2% |
| silica colloidal anhydrous | 0.5% |
| magnesium stearate | 0.5% |
| polyvinylpyrrolidone K30 | 4.1% |
| Coating | 10.3% |

### Process for example 1 and example 2

Process for preparing the delayed release pharmaceutical composition comprising the following steps;
(a)mixing dimethyl fumarate, half of microcrystalline cellulose pH 112, polyvinylpyrrolidone K30, colloidal silicon dioxide for 10 minutes;
(b) sieving half of mg stearat and adding the powder mixture of step (a) then mixing for 3 minutes;
(c) dry granulation of the mixed components;
(d) mixing crospovidone CL and the remaining part microcrystalline cellulose pH 112;
(e) adding this mixture od step (d) in step (c) mixture then mixing for 5 minutes;
(f) sieving the remaining part of mg stearat and adding in step (e) mixture then mixing for 3 minutes,
(g) compressing the blended mixture to form minitablet,
(h) coating with pre-coating (sheffCoatTM Clear ASA) the mini tablet, 30%-35% of all coating comprise, hydroxypropylmethyl cellulose, triacetin/glycerol triacetate, talc,
(i) then coating with enteric coating (sheffCoatTM White ENT ASA) the mini tablet, 45%-55% of all coating which comprising methacrylic acid/ethylacrylate 1:1 co-polymer, talc, titanium dioxide, triethyl citrate,
(j) finally, coating the mini tablet with film-coating (sheffCoatTM Clear ASA) 14%-18% of all coating
(k) filling the minitablets into capsules.

## Claims

1. A delayed release pharmaceutical composition comprising dimethyl fumarate and at least one pharmaceutically acceptable excipient wherein the total amount of dimethyl fumarate in the composition is 18% w/w to 70% w/w.

2. The delayed release pharmaceutical composition according to the claim 1, wherein the total amount of dimethyl fumarate in the composition is 30% to 60% by weight, preferably 35% to 43% by weight.

3. The delayed release pharmaceutical composition according to claim 1 or 2, wherein dimethyl fumarate has a particle size d (0.8) greater than 250 µm, preferably it has a particle size d (0.8) 250 µm to 450 µm, more preferably it has a particle size d (0.8) 280 µm to 450 µm.

4. The delayed release pharmaceutical composition according to any preceding claims, wherein the pharmaceutical composition is in the form of a minitablet.

5. The pharmaceutical composition according to claim 4, wherein the minitablets has at least one coating.

6. The delayed release pharmaceutical composition according to the claim 5, wherein minitablets has at least one film-coating and an enteric coating.

7. The delayed release pharmaceutical composition according to claim 6, wherein the enteric coating layer comprising methacrylic acid/ethylacrylate 1:1 co polymer, talc, titanium dioxide, triethyl citrate or mixtures thereof.

8. The delayed release pharmaceutical composition according to claim 7, wherein the enteric coating layer comprising 50% to 75% by weight of methacrylic acid/ethylacrylate 1:1 co polymer , 15% to 25% by weight of of talc, 5% to 20% by weight of titanium dioxide, 2% to 15% by weight of triethyl citrate or mixtures thereof.

9. The delayed release pharmaceutical composition according to claim 6, wherein the film-coating layer comprising hydroxypropylmethyl cellulose, triacetin/glycerol triacetate, talc or mixtures thereof.

10. The delayed release pharmaceutical composition according to claim 9, wherein film-coating layer comprising 80% to 90% by weight of hydroxypropylmethyl cellulose, 7% to 10% by weight of triacetin/glycerol triacetate, 6% to 8% by weight of talc or mixtures thereof.

11. The delayed release pharmaceutical composition according to the claim 1, wherein at least one pharmaceutically acceptable excipient is selected from the group comprising disintegrants, fillers, binders, glidants, lubricants or mixtures thereof.

12. The delayed release pharmaceutical composition according to the claim 11, wherein the binder is selected from the group comprising polyvinylpyrrolidone, gelatin, sugars, glucose, natural gums, synthetic celluloses, polymethycrylate, hydroxypropyl methyl cellulose, hydroxypropyl cellulose, carboxy methyl cellulose, methyl cellulose or mixtures thereof; preferably the binder is polyvinylpyrrolidone.

13. The delayed release pharmaceutical composition according to the claim 12, wherein the weight ratio of polyvinylpyrrolidone to dimethyl fumarate is 0.01-10.0 , preferably the ratio is 0.05-5.0 , more preferably the ratio is 0.05-1.0 .

14. The delayed release pharmaceutical composition according to any preceding claims, wherein pharmaceutical composition comprising ;
a. 10% w/w and 95% w/w of dimethyl fumarate
b. 30% w/w and 45% w/w of microcrystalline cellulose
c. 3% w/w and 10% w%w of crospovidone
d. 0.3% w/w and 3% w%w of silica colloidal anhydrous
e. 0.1% w/w and 2% w%w of magnesium stearate
f. 1% w/w and 10% w/w of polyvinylpyrrolidone by weight of the total composition.

15. Process for preparing the delayed release pharmaceutical composition according to claim 14 comprising the following steps;
(a) mixing dimethyl fumarate, half of microcrystalline cellulose, polyvinylpyrrolidone, colloidal silicon dioxide for 10 minutes;
(b) sieving half of mg stearat and adding to the powder mixture of step (a) then mixing for 3 minutes;
(c) dry granulation of the mixed components;
(d) mixing crospovidone and the remaining part of microcrystalline cellulose;
(e) adding this mixture of step (d) to step (c) then mixing for 5 minutes;
(f) sieving the remaining part of mg stearat and adding it to step (e) then mixing for 3 minutes,
(g) compressing the blended mixture to form minitablets,
(h) coating with pre-coating the mini tablet, which comprising hydroxypropylmethyl cellulose, triacetin/glycerol triacetate, talc,
(i) then coating with enteric coating the mini tablet , which comprising methacrylic acid/ethylacrylate 1:1 co-polymer, talc, titanium dioxide, triethyl citrate,
(j) finally, coating the mini tablet with film coating,
(k) filling the mini tablets into capsules.
